(19) Europäisches Patentamt
European Patent Office
Office européen des brevets

(11) Publication number: 0 100 641
B1

(12) # EUROPEAN PATENT SPECIFICATION

(45) Date of publication of patent specification: 20.07.88

(51) Int. Cl.[4]: C 12 P 21/00, A 61 K 37/02 // C12N5/00

(21) Application number: 83304310.2

(22) Date of filing: 26.07.83

(54) **Human lymphotoxin and derivatives thereof, process for its preparation, and pharmaceutical composition containing it.**

(30) Priority: 30.07.82 US 403671

(43) Date of publication of application:
15.02.84 Bulletin 84/07

(45) Publication of the grant of the patent:
20.07.88 Bulletin 88/29

(84) Designated Contracting States:
AT BE CH DE FR GB IT LI LU NL SE

(56) References cited:
CHEMICAL ABSTRACTS, vol. 97, no. 19, 8th November 1982, page 532, no. 160840e, Columbus, Ohio, USA, S. PICHYANGKUL et al.: "Purification of lymphotoxin from RPMI-1788 cell line supernate"
CHEMICAL ABSTRACTS, vol. 92, no. 9, 3rd March 1980, page 484, no. 74160y, Columbus, Ohio, USA, M. TOTH et al.: "The human lymphotoxin system. VI. Identification of various saccharides on LT molecules and their contribution to cytotoxicity and charge heterogeneity"

(73) Proprietor: GENENTECH, INC.
460 Point San Bruno Boulevard
South San Francisco California 94080 (US)

(72) Inventor: Aggarwal, Bharat Bhushan
324 Del Rosa Way
San Mateo California 94403 (US)

(74) Representative: Armitage, Ian Michael et al
MEWBURN ELLIS & CO. 2/3 Cursitor Street
London EC4A 1BQ (GB)

(56) References cited:
JOURNAL OF CHROMATOGRAPHY, vol. 235, 29th January 1982, pages 427-433, Elsevier Scientific Publishing Company, Amsterdam, NL., L. FAYE et al.: "Systematic use of affinity differences between immobilized lectin gels for demonstration of glycoprotein molecular variants. The example of radish beta-fructosidase"
CHEMICAL ABSTRACTS, vol. 94, no. 13, 30th March 1981, page 265, no. 97969z, Columbus, Ohio, USA, C.H. EVANS et al.: "Lymphotoxin: an anticarcinogenic lymphokine as measured by inhibition of chemical carcinogen or ultraviolet-irradiation-induced transformation of Syrian hamster cells"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 100 641 B1

Courier Press, Leamington Spa, England.

**Description**

Background of the invention

The invention herein concerns lymphokines, a class of proteins originally extracted in impure form from lymphocytes, and believed to have anti-tumor activity. Specifically, the invention concerns a homogeneous preparation of the human lymphokine, lymphotoxin, and a process for preparing it.

Lymphokines are biologically active, hormone-like peptides or proteins produced by stimulated lymphocytes. The properties and functions of these lymphokines including those exhibiting cytotoxic activity have been studied extensively. Lymphotoxin is a type of lymphokine which is produced not only by mitogen or antigen stimulated lymphocytes, but also by cell lines which are grown in tissue culture derived therefrom.

Lymphotoxin has been implicated in the regulation of the immune system (1) and has been reported to inhibit tumor cell growth both *in vivo* (2—7) and *in vitro* (8—10). Under *in vitro* conditions, it is a more potent inhibitor of tumor cells than of normal ones from the same species (11—15). Also, lymphotoxin preparations have been shown to inhibit UV or chemical carcinogen induced cell transformations (16—17). The *in vitro* activity of lymphotoxin may be assayed by a number of methods as will be set forth hereinbelow and these methods provide convenient means for following the processes of purification of lymphotoxin from crude extracts. *In vivo* studies in human beings have also shown that crude preparations of lymphotoxin are effective in tumor regression (5,7).

Previous studies have been conducted with relatively impure fractions of cell supernatants prepared from lymphocytes or cell lines derived therefrom. A number of laboratories have attempted to prepare lymphotoxin in purified form, without conspicuous success (18—24). The present invention uniquely provides a homogeneous preparation of the human lymphotoxin protein useful as an anti-tumor agent in mammals.

Summary of the invention

In one aspect, the invention is directed to human lymphotoxin per se and its natural and otherwise equivalent pharmaceutically acceptable salts and pharmaceutically acceptable derivatives, in substantially homogeneous form. Homogeneity is defined in its classical sense including the substantial absence of other proteins of human origin. Further, human lymphotoxin is characterized by the sequence of amino acids at the N-terminal end.

The invention further concerns pharmaceutical compositions containing the aforesaid lymphotoxin, and methods of using same for administration as an anti-tumor agent.

In still another aspect, the invention concerns a process for preparing homogeneous human lymphotoxin.

Brief description of the drawings

Figure 1 is a schematic representation of a preferred embodiment of the purification of lymphotoxin as practiced by the invention.

Figure 2 shows the elution pattern from a DEAE cellulose (DE-52) purification step, using 0—0.3 M NaCl gradient. The activity elutes at approximately 0.1 M, while large amounts of protein are retained.

Figure 3 is the isoelectric focusing pattern obtained in a typical preparation.

Figure 4 is the elution pattern from the lentil lectin purification step. More than 95% of the total protein with no lymphotoxin activity is filtered through the column prior the elution of the lymphotoxin.

Figure 5 depicts the elution profile of lymphotoxin activity from Sephadex G-100. The insert shows the calibration curve using the known molecular weight proteins: soybean trypsin inhibitor, carbonic anhydrase, ovalbumin, bovine serum, albumin. Blue dextran is used to determine a size maximum.

Figure 6 shows the results of HPLC used to analyze the purified lymphotoxin fraction from the lentil lectin column.

Figure 7 is the size distribution of the lentil lectin active fraction as determined by SDS-PAGE. A standard using the 20 K fraction from the gel filtration step of Figure 5 is also included.

Figure 8 is the elution profile of lymphotoxin activity from SDS-PAGE of Figure 7. The upper panel shows the results using the gel in column C of Figure 7. The lower panel is a control using only buffer (column E of Figure 7). Slight activity appears to be an artifact associated with the dye front.

Figure 9 shows an analysis of the Rf 0.33 fraction obtained by native-PAGE on purified lymphotoxin by activity determination and by SDS-PAGE. The lowest panel shows the origin of the gel slices from native-PAGE run on the purified lymphotoxin. These slices were assayed both for activity (upper panel) and size (middle panel).

Figure 10 is the elution pattern from HPLC of a trypic digest of the homogeneous lymphotoxin.

Figure 11 is the SDS-PAGE molecular weight analysis of the fractions shown in Figure 10.

Figure 12 shows the results of amino acid sequencing of the fragments from trypsin-digest of purified lymphotoxin.

Detailed description

A. Definitions and abbreviations

The sequence of amino acids in lymphotoxin will be designated by using the standard IUPAC three letter abbreviations for amino acid "residues". "Residue" has a functional meaning within the amino acid sequence. In the body of the sequence the residue and the abbreviation therefor denotes the amino acid less one N-hydrogen and the —OH from the carboxyl; at a terminal position, the designation denotes this residue with the appropriate hydrogen or hydroxide. Thus, in the sequence, $Ala_1$—$Ala_2$—$Ala_3$,

$Ala_1$ denotes 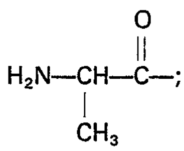 ; $Ala_2$ denotes 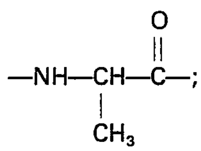 ; $Ala_3$ denotes 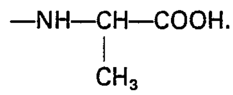 .

All chiral amino acid residues herein are of the natural or L-configuration unless otherwise noted. All peptide sequences are written according to the convention whereby the N-terminal acid is on the left, and the C-terminal on the right.

"Lymphotoxin" apparently can exist in the form of an isolated peptide sequence or in aggregations of several of these peptide chains. The existence of trimers has been inferred from the results obtained herein, and other aggregations may exist under certain conditions. As used herein, "lymphotoxin" includes both the single peptide chain and biologically active aggregations thereof.

"Peptide" and "protein" are used interchangeably herein to denote amino acid polymers; the conventional size distinctions wherein proteins are large and peptides small is not made, in view of the difficulty of finding agreement as to the boundary between the two.

"Amino acid residue" in general refers to a residue as defined hereinabove which is derived from one of the twenty amino acids coded for in proteins. This residue may however be modified so as to form a derivative as defined hereinbelow.

As used herein the term "salts" refers to both salts of carboxyl groups of the polypeptide chain and to acid addition salts of amino groups of the polypeptide chain.

Salts of a carboxyl group may be formed with either inorganic or organic bases by means known in the art per se. Inorganic salts include, for example, sodium, calcium, ammonium, ferric or zinc, salts, and the like. Salts with organic bases include those formed, for example, with amines, such as triethanolamine, arginine or lysine, piperidine, caffeine, procaine, and the like.

Acid addition salts include, for example, salts with mineral acids such as, for example, hydrochloric acid, or sulfuric acid, and salts with organic acids such as, for example, acetic acid, or oxalic acid.

Derivatives may also be prepared from the functional groups which occur as side chains on the residues or the N- or C-terminal groups, by means known in the art, and are included in the invention as long as they remain pharmaceutically acceptable (as defined below). These derivatives may, for example, include:

aliphatic esters of the carboxyl groups;

amides of the carboxyl groups by reaction with ammonia, or with primary or secondary amines;

N-acyl derivatives which are derivatives of an amino group of the polypeptide formed with acyl moieties (e.g. alkanoyl or carboxylic aroyl groups);

or O-acyl derivatives which are derivatives of a hydroxyl group (for example that of seryl or threonyl residues) formed with acyl moieties.

Both the salts and the derivatives encompassed by this invention are those which are "pharmaceutically acceptable" i.e., which do not destroy the activity of the lymphotoxin and which do not confer toxic properties on compositions containing them.

A number of abbreviations with respect to techniques also will be used, which are well known to those skilled in the art. Specifically, "DEAE cellulose chromatography" refers to chromatography using diethylaminoethyl cellulose, which provides an ion exchange support for packing into chromatography columns. At high pH values, the column is an anion exchanger and negative residues stick to column. Elution can be accomplished either by lowering the pH, or, more preferably, with a salt gradient at constant pH.

"PAGE" is electrophoresis as performed on a polyacrylamide gel and separates proteins or peptides on the basis of charge. If sodium dodecyl sulfate (SDS) is incorporated into the gel (SDS-PAGE), the surface active nature of SDS results in a uniform negative charge on the peptide or protein which is a function of size. The result is that separation is based on the molecular size. Native-PAGE denotes the employment of this technique without the presence of SDS and thus, proteins are separated on the basis of charge.

"Isoelectric focusing" can be performed as a form of native PAGE wherein a pH gradient is maintained across the electrodes, causing each protein to stop, or "focus" at its isoelectric point. However, other supports, preferably, for example, dextran, typically Ultrodex-LKB are also practical.

Other chromatography supports are also available which are of particular interest for this invention. A lectin isolated from lentils, "lentil lectin" is capable of retaining, selectively, glycoproteins which have

galactosyl and mannosyl residues. Elution is accomplished by providing a sugar solution; in the case of this particular support, the solution must be of mannose or galactose.

Under suitable circumstances, chromatographic procedures may be carried out preferably in a narrow bore column containing a fine particle resin under increased pressure to enhance the effectiveness of separation, i.e., by high pressure liquid chromatography (HPLC).

Concentration and salt removal are commonly used precursors to certain chromatographic or separation techniques employed in the invention. Salt removal may be performed by, for example, dialysis or gel filtration, or by a relatively recently developed technique, controlled pore glass (CPG).

A number of gel filtration and concentration techniques are also used. Certain commercially available materials are especially useful. "Pellicon" (Trade Mark) membrane is a sheet like material composed of polysulfone manufactured by Millipore, Inc. Bedford; and "Amicon" (Trade Mark) membrane is a similar material also composed of polysulfone and manufactured by Amicon. These materials are capable of retaining large molecules while permitting passage of smaller ones. They thus operate in the opposite way to molecular sieves, which allow large molecules to pass readily but retard the passage of smaller ones. Both Pellicon and Amicon are useful as concentration tools, permitting the smaller molecules to be "filtered" away from the desired macromolecular structures.

The extent to which concentration of a solution to be chromatographed is desirable is largely a matter of practicability of application. Salt removal is necessary if ion exchange or other techniques which depend on total ionic strength are employed. These preparation methods and the extent to which they are required for particular separation procedures are well known in the art.

The cells used as the source of lymphotoxin in the present invention are lymphocytes or their transformants. The literature shows lymphotoxin to be present in preparations of "buffy coat white blood cells" converted by mineral oil induction; golden Syrian hamster or guinea pig peritoneal leukocytes (17) and lymphoblasts such as the human lymphoid cell lines RPMI 1788, B-21 and MOLT (25).

"Specific Activity" refers to the activity of the protein in standard lymphotoxin assays as related to the amount of protein by weight in the sample. As specified in the current disclosure, the activity of lymphotoxin is measured in terms of "units" which refer to the amount of lymphotoxin required to cause 50% lysis in target cells according to the assay procedure set forth hereinbelow. Several standard assay procedures are available. The assay procedure described herein in detail is based on ability to mediate lysis of mouse fibroblast cells, as measured by staining capability.

An additional procedure involves the release of the tritium from tritiated thymidine labeled murine alpha-L-929-fibroblast cells, a lymphotoxin sensitive cell strain (26). The "units" used to define specific activity may differ depending on assay procedure as does the mg protein determined. "Specific activity" as defined herein is units/mg protein, where "units" are measured by the assay set forth below, and mg protein is measured by the method of Bradford (infra).

"Impurities" as they pertain to the lymphotoxin prepared by the method of this invention refers to those substances associated with lymphotoxin in its normal cellular environment or in a crude extract, filtrate, or centrifugate.

B. Preferred embodiment of purification process

The overall process for preparation of homogeneous lymphotoxin, illustrated in the preferred embodiment set forth as in the example below is summarized as Figure 1.

(1) Tissue culture and harvest

The human lymphoblastoid cell line RPMI 1788 was obtained from ATCC (No. CCL 156) and the seed culture was grown in 400 ml of the medium RPMI-1640, obtained from Irvine Scientific, Santa Ana, California, containing 10 mM HEPES and 5% fetal calf serum innoculated at a cell density of $6\times10^4$ cells/ml with 400 mls in 2 liter roller bottles. After 5 days at 37°C, when culture reaches a cell density of $2\times10^6$ ml, the cells were harvested and washed 2 times with a serum-free culture medium RPMI-1640. The cells were then transferred into a serum-free medium RPMI-1640 containing 10 mM HEPES and one percent Penicillin-Streptomycin at a cell density of $5\times10^5$ cells/ml. (The absence of serum in this medium is helpful in the purification of lymphotoxin, as the amount of contaminating protein is decreased). 400 ml of this cell suspension was placed in 2 liter roller bottles and also in 15 liter and 10 liter spinner flasks (Belco Glass Co., Vineland, N.J.), which were substituted for 2 liter roller bottles in some instances. After 65 hours the culture medium was harvested for the lymphotoxin activity.

The cell suspensions from both roller bottles and spinner flasks were pooled. The cells from this pool were removed by passing through a 3 uM Sealkleen filter supplied by Pall Trinity Micro Corp., Cortland, New York (Step 1 of Figure 1). The clear filtrate was further concentrated and dialyzed, as follows (Step 2).

(2) Concentration and dialysis of filtrates

The filtrates were concentrated approximately 20 fold at 4°C on a Pellicon membrane (supplied by Millipore Company) with a molecular weight cut-off of 10,000. The concentrated samples were further dialyzed against 5 mM phosphate buffer, pH 7.8 using the same Pellicon, set up in its dialysis mode. When the conductivity of the sample reached that of the dialysis buffer, the Pellicon membrane was rinsed thoroughly with the buffer and all the protein recovered.

The resulting concentrate was assayed for lymphotoxin activity as were the resultants from Steps 3 through 6, in order to determine specific activity. The specific activity of the concentrate (I) Figure 1 in a typical run was found to be of the order 2,000—3,000 units per mg of protein. Chromatography on a Sephacryl S-300 column to determine molecular size gave a single peak of activity at approximately 70,000 daltons.

(3) DEAE cellulose chromatography

The concentrate (I) was subjected to DEAE cellulose chromatography on a DEAE-52 cellulose column equilibrated in 5 mM phosphate buffer pH 7.8 (Step 3). After the sample was loaded, the column was washed with the equilibration buffer and thereafter it was eluted with a 0—0.3 M linear sodium chloride gradient.

The active fraction was identified by selecting the fraction of the highest specific activity as defined above. A single sharp peak of activity eluted at round 0.1 M NaCl as shown in Figure 2. Typically, this active fraction (I) of Figure 1 would have a specific activity of the order of 20,000—30,000 units per mg protein, a 10 fold purification.

(4) Isoelectric focusing

The active fraction is then concentrated using an Amicon stir cell with 10,000 (PM-10) molecular weight cut-off membrane, (Step 4 of Figure 1).

The concentrate (II) was dialyzed against 2.5 mM Tris and 20 mM glycine pH 8.2 and run on a dextran flat bed (Ultrodex-LKB) containing Ampholine carrier ampholytes in the pH range 5—8 (Step 5). The basic apparatus used for preparative isoelectric focusing was that supplied by LKB (2117 Multiphor). The sample was mixed with the whole gel bed before the pH gradient was formed. After placing the electrode strips on either side of the gel bed, the electrofocusing was run along its length with a constant power of 8W for 15—20 hours at 8°C. The focused zones were collected by sectioning the gel bed with a fractionating grid and then removing each section with a spatula. Elution of the protein from the respective gel section was achieved in several small disposable columns using 50 mM ammonium bicarbonate. The absorbance at 280 nm, lymphotoxin activity, and the pH of the eluted fractions were determined.

Figure 3 shows the activity, protein and pH profile obtained. Though proteins were spread in the whole gel bed, more protein contaminants are observed in the acidic region as compared to basic pH range. The lymphotoxin activity was spread through the pH range of 5.5 to 6.5. Due to the presence of Ampholines it was difficult to determine accurately the protein concentration of the active fraction, thus it was not possible to calculate the degree of purification obtained at the isoelectric focusing step. The recovery of activity from this step was, however, fairly quantitative.

(5) Lentil lectin chromatography

The pool of the isoelectric focusing eluates containing lymphotoxin activity was applied on a Lentil Lectin (Pharmacia) column equilibrated in 10 mM phosphate buffer pH 7.8 (Step 6). After loading the sample the column was washed with the equilibration buffer and then eluted with 50 mM or 100 mM alpha-methylmannoside solution prepared in 10 mM phosphate buffer. The results are shown in Figure 4. More than 95% of the protein, lacking in lymphotoxin activity, filtered through the column initially, and washing with column equilibration buffer (10 mM phosphate buffer pH 7.8) also did not elute the activity. When the column was exposed to 50—100 mM alpha-methylmannoside, a large peak of activity eluted with very little protein. The recovery of units of activity in this step was greater than 90 percent. The active fraction had specific activity on the order of 10 to 100 million units per mg.

C. Characterization of purified lymphotoxin

The active fraction eluted from the Lentil Lectin column was further subjected to the following analytical techniques and has the following properties:

(1) Gel filtration

Gel permeation chromatography gave a single peak at 64,000 daltons, (as shown in Figure 5). The active fraction from the Lentil Lectin column was applied to a Sephadex G-100 column (Pharmacia Fine Chemicals, Piscataway, N.J.), previously equilibrated with the eluting solvent, 10 mM phosphate buffer containing 0.5 M in NaCl. The elution pattern was calibrated with bovine serum albumin, ovalbumin, carbonic anhydrase and soybean trypsin inhibitor; the elution of the lymphotoxin in the 64,000 dalton range indicates aggregation of individual peptides, as will be apparent from the results below.

(2) HPLC Chromatography

HPLC chromatography showed a substantially homogeneous preparation, as shown in Figure 6.

The active fraction from the Lentil Lectin column was concentrated on an Amicon stir cell using PM-10 membrane and applied on a Synchropak RP-P column (25 cm×4.1 mm, Synchrom, Inc., Linden, Indiana). The effluent was monitored at 210 nm and at 280 nm absorbances using a Spectra Physics SP 8000 high pressure liquid chromatograph. The elution conditions consisted of a linear gradient from 0.1% trifluoroacetic acid to 70% acetonitrile in 0.1% trifluoroacetic acid at 25°C and a flow rate of one ml per

minute. A major protein peak which elutes at a concentration of about 50% acetonitrile is obtained (Figure 6) and when run on 12.5% SDS-PAGE, this fraction contains protein with a molecular weight of 20,000. Besides this 20K peak on HPLC, there were also minor peaks which eluted at 43% and 51% acetonitrile with molecular weights of 15K and 70K respectively. The HPLC solvents appear to inactivate the lymphotoxin molecule, thus it was not possible to determine the activity of the 20,000 molecular weight band.

(3) SDS-PAGE

SDS-PAGE showed the majority of the protein to conform to a size estimate of approximately 20,000 daltons (Figure 7). The lymphotoxin activity was associated with this 20,000 dalton fraction, as shown in Figure 8. The SDS-PAGE was performed according to the method of Laemmli, et al. (27), which is incorporated herein by reference, using resolving acrylamide gel concentration of 12.5—15%.

(4) Native-PAGE

Native-PAGE also gave a peak of approximate MW of 20,000, as subsequently determined by SDS-PAGE.

The active fraction from the Lentil Lectin column was subjected to the native-PAGE procedure of Laemmli, et al. (27) with slight modifications. Both preparative (1.5—4 mm thick) and analytical (0.75 mm thick) gels consisted of 7.5% acrylamide for resolving gel and 4% for stacking gel. The running buffer used in both cases was Tris 25 mM pH 6.8. The analytical gel was run at constant current of 22 mA and preparative gel was run at 100 mA keeping the temperature constant.

At the end of the electrophoretic run, the protein on the gel was visualized by silver staining (28) incorporated herein by reference, to give a diffused band of Rf of 0.33. Figure 9 gives a more detailed explanation of these results—as there shown, it was determined in a separate experiment that when gels are sliced and eluted by incubating the slices overnight at 4°C in 50 mM ammonium carbonate buffer from the native-PAGE, the lymphotoxin activity is associated with a gel slice of Rf 0.33. The eluates from these gel slices were applied on SDS-PAGE, and the 20,000 MW band appeared only in those eluates which had lymphotoxin activity.

(5) Analysis by tryptic digestion and isolation of peptides

The purified lymphotoxin preparation was digested with trypsin-TPCK (Worthington) using 1 part trypsin, 20 parts protein in 50 mM ammonium bicarbonate buffer pH for 24 hours at room temperature. The fragments have approximate molecular weights of 15,000 and 5,000.

At the end of hydrolysis, the tryptic peptides were chromatographed by HPLC on a Lichrosorb RP-18 column (25 cm×4.6 mm, EM reagents, Cincinnati, Ohio) at 25°C, using Spectra Physics SP-8000 chromatograph. The peaks were detected at 210 nm and 280 nm after elution with a linear gradient of 0.1% trifluoroacetic acid to 50% acetonitrile in 0.1% trifluoroacetic acid at a flow rate of two ml per minute. (For later amino acid sequence analysis, each peak was collected, lyophilized and stored at −20°C). As shown in Figure 10, two peaks, T-1 and T-2, were observed eluting at 42.3% and 48.4% acetonitrile concentration respectively. The molecular size of each of these 2 peaks as determined by SDS-PAGE (Figure 11) was 15,000 for T-1 and 5,000 for T-2. Both at 210 nm and 280 nm, the T-2 peak with relatively lower MW had higher absorbance than the T-1 peak.

The completeness of the trypsin hydrolysis was confirmed by applying a small aliquot of the hydrolyzed preparation to SDS-PAGE and showing that 20,000 dalton band was undetectable, and had been replaced by the 15,000 and 5,000 dalton bands.

Furthermore, the composition formed by the trypsin digestion as herein described exhibits lymphotoxin activity.

(6) Automated amino acid sequence analysis

Both intact and tryptic fragments were sequenced by automatic amino acid sequencing techniques by suspending the samples in 0.6 ml of 0.2 M acetic acid and transferring the suspension into the cup of a Beckman 890C sequencer which had been previously coated with 4 mg polybrene and 100 n moles norleucine. The phenyl thiohydanation (PTH) amino acids were identified using an ultrasphere ODS (25 cm×4.6 mm, 5 uM) column at a flow rate of one ml per minute with a program as described in, Kohr, W. et al (29) incorporated herein by reference.

The results obtained with both the intact protein and the tryptic fragments are shown in Figure 12. As there seen, certain positions in the sequence could not be identified with certainty. However, sufficient identification was made to show that the 15K fragment (T-1) corresponds to the N-terminal portion of the intact lymphotoxin.

The above example is intended to illustrate rather than limit the invention. Other cell lines can be used as starting material; and equivalent chromatographic methods employed. The greatest purification appears to be achieved by the use of lentil lectin chromatography and as long as this step is included, a variety of preliminary steps remain satisfactory. Lentil lectin has not heretofore been used in the isolation of lymphotoxin. However, the integrated process as shown above provides a result which is substantially a pure protein.

D. Assay procedure for lymphotoxin activity

The activity of lymphotoxin during purification was monitored by the modified cell-lytic assay of Spofford (30) which is incorporated herein by reference. Briefly, mouse L-929 fibroblast cells were grown in microtiter plates in the presence of mitomycin C. After 12—18 hours 0.125 ml of serially diluted sample to be assayed for lymphotoxin is added to each well. After 48 hours, the plates were washed and the lysis of the cells induced by lymphotoxin was detected as adhering to the plates by staining the plates with a 1% solution of crystal violet in methanol:water (1:4 v/v). The intensity of stain was observed both visually as well as spectrophotometrically at absorbance of 450 nm and 570 nm transmission using a Dynatech spectrophotometer. The cells plated in a microtiter well with culture medium alone were set at 0% lysis whereas those with 3M guanidine hydrochloride solution provided an end point for 100% lysis. One unit of lymphotoxin is defined as the amount required for 50% cell lysis out of 12,000 cells plated in each well.

E. Determination of protein

The protein concentration throughout purification was determined by the method of Bradford (31) incorporated herein by reference. Briefly, 0.8 ml of the sample to be assayed was mixed with the 0.2 ml of the reagent concentrate solution from Bio-Rad Labs, Richmond, California. The reaction mixture was read at 595 nm in a Perkin Elmer Spectrophotometer Model 55B. This assay was linear between 2—20 μg of bovine serum albumin. During the final stages of purification, a rough estimate of protein was also made either from absorbance at 280 and 206 nm or by the silver staining of the SDS-polyacrylamide gels. This rough estimate was eventually confirmed by the nanomoles of various amino acid residues appearing at each cycle of the protein sequencing.

F. Utility and administration

Administration of the lymphotoxin or its salts or derivatives or its active components can be via any of the accepted modes of administration for agents which exhibit anti-tumor activity. These methods include oral, parenteral or topical administrations and otherwise systemic forms. Local or intravenous injection is preferred. The active components, for example, include the tryptic digest of lymphotoxin which as been shown to be active, and there is no reason it could not be used as the active ingredient in a pharmaceutical composition of this kind and for this purpose.

Depending on the intended mode of administration, the compositions used may be in the form of solid, semi-solid or liquid dosage forms, such as, for example, tablets, pills, capsules, powders, liquids, suspensions, or the like, preferably in unit dosage forms suitable for single administration of precise dosages. The compositions will include a conventional pharmaceutical carrier or excipient the lymphotoxin or the pharmaceutically acceptable salts or derivatives thereof and, in addition, may include other medicinal agents, pharmaceuticals agents, carriers, adjuvants, etc. Such excipients may include other proteins, such as, for example, human serum albumin or plasma preparations.

The amount of active compound administered will of course, be dependent on the subject being treated, the severity of the affliction, the manner of administration and the judgement of the prescribing physician. However, an effective dosage is in the range of 0.007—7 ng/kg/day, preferably 0.07—0.7 ng/kg/day.

For solid compositions, conventional non-toxic solid carriers include, for example, pharmaceutical grades or mannitol, lactose starch, or magnesium stearate. Liquid pharmaceutically administerable compositions can, for example, be prepared by dissolving, dispersing, etc. the lymphotoxin as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of non-toxic auxiliary substances such as wetting or emulsifying agents, pH buffering agents and the like, for example, sodium acetate or sorbitan monolaurate. Actual methods of preparing such dosage forms are known, or will be apparent, to those skilled in this art; for example, see *Remington's Pharmaceutical Sciences*, Mack Publishing Company, Easton, Pennsylvania, 15th Edition, 1975. The composition or formulation to be administered will, in any event, contain a quantity of the lymphotoxin in an amount effective to alleviate the symptoms of the subject being treated.

G. References

The bibliography appended hereto, and referenced in the foregoing text is included to enhance the understanding of the invention. The subject matter so referenced is thereby incorporated herein.

Bibliography

1. Evans, *Cancer Immunology and Immunotherapy 12,* 181 (1982).
2. Holterman, O. A., et al. "Studies on local administration of materials with lymphokine activity to neoplasms involving the skin". In M. A. Fink (ed.). The macrophase in neoplasia. pp. 259—261, *Academic Press, New York* (1976).
3. May-Levin, F., et al. *Schweiz. Med. Wsch,* 102:1188 (1972).
4. Papermaster, B. W., et al. *Res. Comm. Chem. Pah. Pharmacol,* 8:413 (1974).
5. Papermaster, B. W., et al. *Clin. Immunol. Immunopathol,* 5:31 (1976).

6. Papermaster, B. W., et al. *Ann. N.Y. Acad. Sci,* 332:451 (1979).
7. Khan, A., et al. *Hematology and Oncology,* 11:128A (1981).
8. Gately, M. K., et al. *Immunol,* 27:B2 (1976).
9. Rosenberg, S. A., et al. *J. Immunol,* 110:1623 (1973).
10. Swada, J. I., et al. *Jap. J. Med.,* 46:263 (1976).
11. Williams, T. W., et al. *Cell Immunol,* 6:171 (1973).
12. Evans, C. H. et al. *Cancer Res.,* 35:1035 (1975).
13. Meltzer, M. S., et al. *J. Nat. Cancer Inst.,* 49:1439 (1972).
14. Rundell, J. O., et al. *Immunopharmacology,* 3:9 (1981).
15. Weedon, D. D., et al. *Mayo Clinic Pro.,* 48:556 (1973).
16. Evans, C. H., et al. *Cancer Res.,* 37:898 (1977).
17. Evans, C. H., et al. *Int. J. Cancer,* 27:45 (1981).
18. Klostergaard, J., et al. *Immunol,* 18:1049 (1981).
19. Klostergaard, J., et al. *Mol. Immunol,* 18:455 (1981).
20. Pitchyangkil, et al. *J. of Clinical Hematology and Oncology 11,* 19A (1981).
21. Amino, N., et al. *J. Immunol,* 113:1334 (1974).
22. Russell, S. W., et al. *J. Immunol,* 109:784 (1972).
23. Fuhrer, J. P., et al. "Biologically active Syrian hamster lymphotoxin isolated in high yield on a Waters I-125 Protein HPLC column." (1982) In International symptons on HPLC of Proteins and peptides held in Washington, D. C.—November 16—17th, 1981.
24. Klostergaard, J., et al. *Mol. Immunol,* 17:613 (1980).
25. Lisafeld, et al. *Int. Archs. Allergy Appl. Immun.,* 62:59 (1980).
26. Heffes, et al. *J. Immunol,* 14:64 (1975).
27. Laemmli U. K., et al, *Nature,* 227:680 (1970).
28. Merril, C. R., et al. *Anal Biochem,* 110:201 (1981).
29. Kohr, W., et al. *Anal Biochem* (in press).
30. Spofford, B., et al. *J. Immunol,* 112:2111 (1974).
31. Bradford, N. M., et al. *Anal Brochem,* 72:248 (1976).

**Claims for the Contracting States: BE, CH, DE, FR, GB, IT, LI, LU, NL, SE**

1. A process for preparing human lymphotoxin, which process comprises:
(a) obtaining an extract, supernatant or filtrate from a cell culture which produces lymphotoxin;
(b) treating said extract, supernatant or filtrate with a lentil lectin absorbant;
(c) selectively eluting the lymphotoxin from the absorbant.

2. The process of claim 1 which includes, before step (b) the additional step of isoelectric focusing.

3. The process of claim 1 or claim 2 which includes, before or after the step of isoelectric focusing (if present), the additional step of DEAE-cellulose chromatography.

4. A process for preparing lymphotoxin, which process comprises:
(a) concentrating the extract, supernatant or filtrate from a cell culture which produces lymphotoxin and dialyzing the concentrate to obtain a dialyzate;
(b) subjecting the dialyzate to DEAE-cellulose chromatography;
(c) subjecting the fraction containing lymphotoxin from step (b) to concentration;
(d) subjecting the resultant of (c) to isoelectric focusing to obtain a fraction containing lymphotoxin.
(e) subjecting the fraction containing lymphotoxin from step (d) to lentil lectin chromatography;
(f) recovering lymphotoxin from step (e).

5. The process of claim 4 wherein the dialysis in step (a) is performed using a polysulfone sheet.

6. The process of claim 4 or claim 5 wherein the lymphotoxin in step (f) is obtained by selectively eluting with a solution containing mannose or galactose.

7. The process of any one of the preceding claims wherein the cell culture is of a human lymphoblastoid cell line.

8. The process of claim 7 wherein the cell line is RPMI 1788 (ATCC No. CCL 156).

9. The process of any one of the preceding claims wherein the cell culture has been grown in serum free medium.

10. Human lymphotoxin characterised by having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala - , substantially free of impurities; and salts and derivatives of the amino acid residue side chains or the N- or C-terminal groups.

11. Human lymphotoxin characterised by having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala - , with a specific activity greater than $10\times10^6$ units/mg; and salts and derivatives of the amino acid residue side chains or the N- or C-terminal groups.

12. Human lymphotoxin characterised by having the partial amino acid sequences His - Ser - Thr - Leu - Lys - Pro - Ala - Ala - His - Leu - Ile - Gly - Asp - Pro - Ser - Lys - Gln - Asn - and Ala - Thr -

Ser - Ser - Pro - Leu - Tyr - Leu - Ala - , with a specific activity greater than $100 \times 10^6$ units/mg; and salts and derivatives of the amino acid residue side chains or the N- or C-terminal groups.

13. A protein characterised by having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala - , which is active in standard assays for human lymphotoxin and which is substantially homogeneous with respect to the HPLC performed as described herein in the specification; and salts and derivatives of the amino acid residue side chains or the N- or C-terminal groups.

14. A protein characterised by having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala - , which is active in standard assays for human lymphotoxin and which is substantially homogeneous with respect to SDS-PAGE performed as described herein in the specification; and salts and derivatives of the amino acid residue side chains or the N- or C-terminal groups.

15. Purified underivatised protein of any one of claims 10 to 14.

16. Protein of any one of claims 10 to 15 in pharmaceutically acceptable form.

17. Protein of any one of claims 10 to 15 in non-radioactive form.

18. A composition of matter which comprises a trypic digest of the protein of any one of claims 10 to 17, in which one tryptic fragment has the partial amino acid sequence His - Ser - Thr - Leu - Lys - Pro - Ala - Ala - His - Leu - Ile - Gly - Asp - Pro - Ser - Lys - Gln - Asn - and another fragment has the partial amino acid sequence Ala - Thr - Ser - Ser - Pro - Leu - Tyr - Leu - Ala - .

19. A pharmaceutical composition useful for treating tumors in humans which comprises a therapeutically effective amount of a compound of any one of claims 10 to 18 in admixture with a pharmaceutically acceptable excipient.

**Claims for the Contracting State: AT**

1. A process for preparing human lymphotoxin, which process comprises:

(a) obtaining an extract, supernatant or filtrate from a cell culture which produces lymphotoxin;

(b) treating said extract, supernatant or filtrate with a lentil lectin absorbant;

(c) selectively eluting the lymphotoxin from the absorbant.

2. The process of claim 1 which includes, before step (b) the additional step of isoelectric focusing.

3. The process of claim 1 or claim 2 which includes, before or after the step of isoelectric focusing (if present), the additional step of DEAE-cellulose chromatography.

4. A process for preparing lymphotoxin, which process comprises:

(a) concentrating the extract, supernatant or filtrate from a cell culture which produces lymphotoxin and dialyzing the concentrate to obtain a dialyzate;

(b) subjecting the dialyzate to DEAE-cellulose chromatography;

(c) subjecting the fraction containing lymphotoxin from step (b) to concentration;

(d) subjecting the resultant of (c) to isoelectric focusing to obtain a fraction containing lymphotoxin.

(e) subjecting the fraction containing lymphotoxin from step (d) to lentil lectin chromatography;

(f) recovering lymphotoxin from step (e).

5. The process of claim 4 wherein the dialysis in step (a) is performed using a polysulfone sheet.

6. The process of claim 4 or claim 5 wherein the lymphotoxin in step (f) is obtained by selectively eluting with a solution containing mannose or galactose.

7. The process of any one of the preceding claims wherein the cell culture is of a human lymphoblastoid cell line.

8. The process of claim 7 wherein the cell line is RPMI 1788 (ATCC No. CCL 156).

9. The process of any one of the preceding claims wherein the cell culture has been grown in serum free medium.

10. A process which comprises producing human lymphotoxin having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala - , substantially free of impurities; and salts and derivatives of the amino acid residue side chains or the N- or C-terminal groups.

11. A process which comprises producing human lymphotoxin having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala - , with a specific activity greater than $10 \times 10^6$ units/mg; and salts and derivatives of the amino acid residue side chains or the N- or C-terminal groups.

12. A process which comprises producing human lymphotoxin having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala - , with a specific activity greater than $100 \times 10^6$ units/mg; and salts and derivatives of the amino acid residue side chains or the N- or C-terminal groups.

13. A process which comprises producing a protein having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala - , which is active in standard assays for human

lymphotoxin and which is substantially homogeneous with respect to the HPLC performed as described herein in the specification; and salts and derivatives of the amino acid residue side chains or the N- or C-terminal groups.

14. A process which comprises producing a protein having the partial amino acid sequences His - Ser - Thr - Leu - Lys - Pro - Ala - Ala - His - Leu - Ile - Gly - Asp - Pro - Ser - Lys - Gln - Asn - and Ala - Thr - Ser - Ser - Pro - Leu - Tyr - Leu - Ala - , which is active in standard assays for human lymphotoxin and which is substantially homogeneous with respect to SDS-PAGE performed as described herein in the specification; and salts and derivatives of the amino acid residue side chains or the N- or C-terminal groups.

15. A process of any one of claims 10 to 14 wherein the protein is produced in purified, underivatised form.

16. A process of any one of claims 10 to 15 wherein the protein is produced in pharmaceutically acceptable form.

17. A process of any one of claims 10 to 15 wherein the protein is produced in non-radioactive form.

18. A process which comprises producing a composition of matter by tryptic digestion of the protein of any one of claims 10 to 17, in which one tryptic fragment has the partial amino acid sequence His - Ser - Thr - Leu - Lys - Pro - Ala - Ala - His - Leu - Ile - Gly - Asp - Pro - Ser - Lys - Gln - Asn - and another fragment has the partial amino acid sequence Ala - Thr - Ser - Ser - Pro - Leu - Tyr - Leu - Ala - .

**Patentansprüche für die Vertragsstaaten: BE, CH, DE, FR, GB, IT, LI, LU, NL, SE**

1. Verfahren zur Herstellung von menschlichem Lymphotoxin, welches Verfahren umfaßt:

(a) Erhalten eines Extraktes, Überstandes oder Filtrates aus einer Zellkultur, die Lymphotoxin produziert;

(b) Behandeln des genannten Extraktes, Überstandes oder Filtrates mit einem Linsenlectin-Adsorbens;

(c) selektives Eluieren des Lymphotoxins aus dem Adsorbens.

2. Verfahren nach Anspruch 1, das vor dem Schritt (b) den zusätzlichen Schritt des isoelektrischen Fokussierens umfaßt.

3. Verfahren nach Anspruch 1 oder 2, das vor oder nach dem Schritt des isoelektrischen Fokussierens (falls dies vorgenommen wird), den zusätzlichen Schritt der DEAE-Cellulose-Chromatographie umfaßt.

4. Verfahren zur Herstellung von Lymphotoxin, welches Verfahren umfaßt:

(a) Konzentrieren des Extraktes, Überstandes oder Filtrates aus einer Zellkultur, die Lymphotoxin produziert, und Dialysieren des Konzentrates, um ein Dialysat zu erhalten;

(b) Unterwerfen des Dialysates der DEAE-Cellulose-Chromatographie;

(c) Konzentrieren der Lymphotoxin enthaltenden, aus Schritt (b) herrührenden Fraktion;

(d) isoelektrisches Fokussieren der in (c) erhaltenen Substanz, um eine Lymphotoxin enthaltende Fraktion zu erhalten;

(e) Unterwerfen der aus Schritt (d) herrührenden, Lymphotoxin enthaltenden Fraktion der Linsenlectin-Chromatographie;

(f) Gewinnen des aus Schritt (e) herrührenden Lymphotoxins.

5. Verfahren nach Anspruch 4, worin die Dialyse in Schritt (a) unter Verwendung einer Polysulfon-Folie vorgenommen wird.

6. Verfahren nach Anspruch 4 oder 5, worin das Lymphotoxin in Schritt (f) durch selektives Eluieren mit einer Mannose oder Galactose enthaltenden Lösung erhalten wird.

7. Verfahren nach einem der vorhergehenden Ansprüche, worin die Zellkultur von einer meschlichen Lymphoblastoid-Zellenlinie stammt.

8. Verfahren nach Anspruch 7, worin die Zellenlinie RPMI 1788 (ATCC Nr. CCL 156) ist.

9. Verfahren nach einem der vorhergehenden Ansprüche, worin die Zellkultur in einem serumfreien Medium kultiviert wurde.

10. Menschliches Lymphotoxin, gekennzeichnet durch die Aminosäureteilsequenz His - Ser - Thr - Leu - Lys - Pro - Ala - Ala - His - Leu - Ile - Gly - Asp - Pro - Ser - Lys - Gln - Asn und Ala - Thr - Ser - Ser - Pro - Leu - Tyr - Leu - Ala - , im wesentlichen frei von Verunreinigungen; und Salze und Derivate der Aminosäurerestseitenketten oder der N- oder C-endständigen Gruppen.

11. Menschliches Lymphotoxin, gekennzeichnet durch die Aminosäureteilsequenzen His - Ser - Thr - Leu - Lys - Pro - Ala - Ala - His - Leu - Ile - Gly - Asp - Pro - Ser - Lys - Gln - Asn - und Ala - Thr - Ser - Ser - Pro - Leu - Tyr - Leu - Ala - , mit einer spezifischen Aktivität von mehr als $10\times10^6$ Einheiten/mg; und Salze und Derivate der Aminosäurerestseitenketten oder der N- oder C-endständigen Gruppen.

12. Menschliches Lymphotoxin, gekennzeichnet durch die Aminosäureteilsequenzen His - Ser - Thr - Leu - Lys - Pro - Ala - Ala - His - Leu - Ile - Gly - Asp - Pro - Ser - Lys - Gln - Asn - und Ala - Thr - Ser - Ser - Pro - Leu - Tyr - Leu - Ala - , mit einer spezifischen Aktivität von mehr als $100\times10^6$ Einheiten/mg; und Salze und Derivate der Aminosäurerestseitenketten oder der N- oder C-endständigen Gruppen.

13. Ein Protein, gekennzeichnet durch die Amonisäureteilsequenzen His - Ser- - Thr - Leu - Lys - Pro - Ala - Ala - His - Leu - Ile - Gly - Asp - Pro - Ser - Lys - Gln - Asn - und Ala - Thr - Ser - Ser - Pro -

Leu - Tyr - Leu - Ala - , das aktiv in Standardanalysen für menschliches Lymphotoxin und im wesentlichen homogen in bezug auf die wie in der vorliegenden Beschreibung durchgeführte HPLC-Chromatographie ist; und Salze und Derivate der Aminosäurerestseitenketten oder der N- oder C-endständigen Gruppen.

14. Ein Protein, gekennzeichnet durch die Aminosäureteilsequenzen His - Ser - Thr - Leu - Lys - Pro - Ala - Ala - His - Leu - Ile - Gly - Asp - Pro - Ser - Lys - Gln - Asn - und Ala - Thr - Ser - Ser - Pro - Leu - Tyr - Leu - Ala - , das aktiv in Standardanalysen für menschlches Lymphotoxin und im wesentlichen homogen in bezug auf die wie in der vorliegenden Beschreibung durchgeführtes SDS-PAGE ist; und Salze und Derivate der Aminosäurerestseitenketten oder der N- oder C-endständigen Gruppen.

15. Gereinigtes, underivatisiertes Protein nach einem der Ansprüche 10 bis 14.

16. Protein nach einem der Anuprüche 10 bis 15 in pharmazeutisch verträglicher Form.

17. Protein nach einem der Ansprüche 10 bis 15 in nichtradioaktiver Form.

18. Eine Zusammensetzung aus Material, die einen tryptischen Digest des Proteins nach einem der Ansprüche 10 bis 17 umfaßt, in der ein tryptisches Fragment die Aminosäureteilsequenz His - Ser - Thr - Leu - Lys - Pro - Ala - Ala - His - Leu - Ile - Gly - Asp - Pro - Ser - Lys - Gln - Asn - und ein anderes Fragment die Aminosäureteilsequenz Ala - Thr - Ser - Ser - Pro - Leu - Tyr - Leu - Ala - aufweist.

19. Eine pharmazeutische Zusammensetzung, die nützlich für die Behandlung von Tumoren beim Menschen ist und eine therapeutisch wirksame Menge einer Verbindung nach einem der Ansprüche 10 bis 18 vermischt mit einem pharmazeutisch verträglichen Excipiens enthält.

**Patentansprüche für den Vertragsstaat: AT**

1. Verfahren zur Herstellung von menschlichem Lymphotoxin, welches Verfahren umfaßt:

(a) Erhalten eines Extraktes, Überstandes oder Filtrates aus einer Zellkultur, die Lymphotoxin produziert;

(b) Behandeln des genannten Extraktes, Überstandes oder Filtrates mit einem Linsenlectin-Adsorbens;

(c) selektives Eluieren des Lymphotoxins aus dem Adsorbens.

2. Verfahren nach Anspruch 1, das vor dem Schritt (b) den zusätzlichen Schritt des isoelektrischen Fokussierens umfaßt.

3. Verfahren nach Anspruch 1 oder 2, das vor oder nach dem Schritt des isoelektrischen Fokussierens (falls dies vorgenommen wird), den zusätzlichen Schritt der DEAE-Cellulose-Chromatographie umfaßt.

4. Verfahren zur Herstellung von Lymphotoxin, welches Verfahren umfaßt:

(a) Konzentrieren des Extraktes, Überstandes oder Filtrates aus einer Zellkultur, die Lymphotoxin produziert, und Dialysieren des Konzentrates, um ein Dialysat zu erhalten;

(b) Unterwerfen des Dialysates der DEAE-Cellulose-Chromatographie;

(c) Konzentrieren der Lymphotoxin enthaltenden, aus Schritt (b) herrührenden Fraktion;

(d) isoelektrisches Fokussieren der in (c) erhaltenen Substantz, um eine Lymphotoxin enthaltende Fraktion zu erhalten;

(e) Unterwerfen der aus Schritt (d) herrührenden, Lymphotoxin enthaltenden Fraktion der Linsenlectin-Chromatographie;

(f) Gewinnen des aus Schritt (e) herrührenden Lymphotoxins.

5. Verfahren nach Anspruch 4, worin die Dialyse in Schritt (a) unter Verwendung einer Polysulfon-Folie vorgenommen wird.

6. Verfahren nach Anspruch 4 oder 5, worin das Lymphotoxin in Schritt (f) durch selektives Eluieren mit einer Mannose oder Galactose enthaltenden Lösung erhalten wird.

7. Verfahren nach einem der vorhergehenden Ansprüche, worin die Zellkultur von einer menschlichen Lymphoblastoid-Zellenlinie stammt.

8. Verfahren nach Anspruch 7, worin die Zellenlinie RPMI 1788 (ATCC Nr. CCL 156) ist.

9. Verfahren nach einem der vorhergehenden Ansprüche, worin die Zellkultur in einem serumfreien Medium kultiviert wurde.

10. Verfahren, umfassend die Herstellung von menschlichem Lymphotoxin mit den Aminosäureteilsequenzen His - Ser - Thr - Leu - Lys - Pro - Ala - Ala - His - Leu - Ile - Gly - Asp - Pro - Ser - Lys - Gln - Asn und Ala - Thr - Ser - Ser - Pro - Leu - Tyr - Leu - Ala - , im wesentlichen frei von Verunreinigungen; und Salzen und Derivaten der Aminosäurerestseitenketten oder der N- oder C-endständigen Gruppen.

11. Verfahren, umfassend die Herstellung von menschlichem Lymphotoxin mit den Aminosäureteilsequenzen His - Ser - Thr - Leu - Lys - Pro - Ala - Ala - His - Leu - Ile - Gly - Asp - Pro - Ser - Lys - Gln - Asn - und Ala - Thr - Ser - Ser - Pro - Leu - Tyr - Leu - Ala - , mit einer spezifischen Aktivität von mehr als $10\times10^6$ Einheiten/mg; und Salzen und Derivaten der Aminosäurerestseitenketten oder der N- oder C-endständigen Gruppen.

12. Verfahrend, umfassend die Herstellung von menschlichem Lymphotoxin mit den Aminosäureteilsequenzen His - Ser - Thr - Leu - Lys - Pro - Ala - Ala - His - Leu - Ile - Gly - Asp - Pro - Ser - Lys - Gln - Asn - under Ala - Thr - Ser - Ser - Pro - Leu - Tyr - Leu - Ala - , mit einer spezifischen Aktivität von mehr als $100\times10^6$ Einheiten/mg; und Salzen und Derivaten der Aminosäurerestseitenketten oder der N- oder C-endständigen Gruppen.

13. Verfahren, umfassend die Herstellung eines Proteins mit den Aminosäureteilsequenzen His - Ser - Thr - Leu - Lys - Pro - Ala - Ala - His - Leu - Ile - Gly - Asp - Pro - Ser - Lys - Gln - Asn - und Ala - Thr - Ser - Ser - Pro - Leu - Tyr - Leu - Ala - , das aktiv in Standardanalysen für menschliches Lymphotoxin und im wesentlichen homogen in bezug auf die wie in der vorliegenden Beschreibung durchgeführte HPLC-Chromatographie ist; und von Salzen und Derivaten der Aminosäurerestseitenketten oder der N- oder C-endständigen Gruppen.

14. Verfahren, umfassend die Herstellung eines Proteins mit den Aminosäureteilsequenzen His - Ser - Thr - Leu - Lys - Pro - Ala - Ala - His - Leu - Ile - Gly - Asp - Pro - Ser - Lys - Gln - Asn - und Ala - Thr - Ser - Ser - Pro - Leu - Tyr - Leu - Ala - , das aktiv in Standardanalysen für menschliches Lymphotoxin und im wesentlichen homogen in bezug auf die wie in der vorliegenden Beschreibung durchgeführte SDS-PAGE ist; und von Salzen und Derivaten der Aminosäurerestseitenketten oder der N- oder C-endständigen Gruppen.

15. Verfahren nach einem der Ansprüche 10 bis 14, worin das Protein in gereinigter, underivatisierter Form hergestellt wird.

16. Verfahren nach einem der Ansprüche 10 bis 15, worin das Protein in pharmazeutisch verträglicher Form hergestellt wird.

17. Verfahren nach einem der Ansprüche 10 bis 15, worin das Protein in nichtradioaktiver Form hergestellt wird.

18. Verfahren, umfassend die Herstellung einer Zusammensetzung aus Material durch tryptische Digestion des Proteins nach einem der Ansprüche 10 bis 17, in dem ein tryptisches Fragment die Aminosäureteilsequenzen His - Ser - Thr - Leu - Lys - Pro - Ala - Ala - His - Leu - Ile - Gly - Asp - Pro - Ser - Lys - Gln - Asn - und ein anderes Fragment die Aminosäureteilsequenzen Ala - Thr - Ser - Ser - Pro - Leu - Tyr - Leu - Ala - aufweist.

**Revendications pour les Etats Contractants: BE, CH, DE, FR, GB, IT, LI, LU, NL, SE**

1. Procédé pour la préparation de lymphotoxine humaine, lequel procédé consiste à:

(a) obtenir un extrait, produit surnageant ou filtrat d'une culture de cellules qui produit la lymphotoxine;

(b) traiter ledit extrait, produit surnageant ou filtrat au moyen d'un absorbant de lectine de lentille;

(c) éluer sélectivement la lymphotoxine de l'absorbant.

2. Procédé de la revendication 1 qui comprend, avant l'étape (b), l'étape additionnelle de focalisation isoélectrique.

3. Procédé de la revendication 1 ou la revendication 2 qui comprend, avant ou après létape de focalisation isoélectrique (si elle est présente), l'étape additionnelle de chromatographie sur DEAE-cellulose.

4. Procédé de préparation de lymphotoxine, lequel procédé consiste à:

(a) concentrer l'extrait, produit surnageant ou filtrat d'une culture de cellules qui produit la lymphotoxine et dialyser le concentrat pour obtenir un dialysat;

(b) soumettre le dialysat à une chromatographie sur DEAE-cellulose;

(c) soumettre la fraction contenant la lymphotoxine de l'étape (b) à une concentration;

(d) soumettre la résultat de (c) à une focalisation isoélectrique pour obtenir une fraction contenant la lymphotoxine;

(e) soumettre la fraction contenant la lymphotoxine de l'étape (d) à une chromatographie à la lectine de lentille;

(f) récupérer la lymphotoxine de l'étape (e).

5. Procédé de la revendication 4 où la dialyse à l'étape (a) est accomplie en utilisant une feuille de polysulfone.

6. Procédé de la revendication 4 ou la revendication 5 où la lymphotoxine de l'étape (f) est obtenue en éluant sélectivement avec une solution contenant du mannose ou du galactose.

7. Procédé selon l'une quelconque des revendications précédentes où la culture de cellules est d'une lignée de cellules de lymphoblastoîde humain.

8. Procédé selon la revendication 7 où la lignée de cellules est RPMI 1788 (ATCC No. CCL 156).

9. Procédé selon l'une quelconque des revendications précédentes où la culture de cellules a été développée dans un milieu sans sérum.

10. Lymphotoxine humaine caractérisée en ce qu'elle a les séquences partielles d'acides aminés His - Ser - Thr - Leu - Lys - Pro - Ala - Ala - His - Leu - Ile - Gly - Asp - Pro - Ser - Lys - Gln - Asn - et Ala - Thr - Ser - Ser - Pro - Leu - Tyr - Leu - Ala - , sensiblement sans impuretés; et les sels et dérivés des chaînes secondaires de résidus d'acide aminé ou les groupes N- ou C-terminaux.

11. Lymphotoxine humaine charactérisée en ce qu'elle a les séquences partielles d'acides aminés His - Ser - Thr - Leu - Lys - Pro - Ala - Ala - His - Leu - Ile - Gly - Asp - Pro - Ser - Lys - Gln - Asn - et Ala - Thr - Ser - Ser - Pro - Leu - Tyr - Leu - Ala - , avec une activité spécifique supérieure à $10 \times 10^6$ unités/mg; et les sels et dérivés des chaînes secondaires de résidus d'acide aminé ou les groupes N- ou C-terminaux.

12. Lymphotoxine humaine caractérisée en ce qu'elle a les séquences partielles d'acides aminés His - Ser - Thr - Leu - Lys - Pro - Ala - Ala - His - Leu - Ile - Gly - Asp - Pro - Ser - Lys - Gln - Asn - et Ala - Thr - Ser - Ser - Pro - Leu - Tyr - Leu - Ala - , une activité spécifique supérieure à $100 \times 10^6$ unités/mg; et les sels et dérivés des chaînes secondaires de résidus d'acide aminé ou les groupes N- ou C-terminaux.

13. Protéine caractérisée en ce qu'elle a les séquences partielles d'acides aminés His - Ser - Thr - Leu - Lys - Pro - Ala - Ala - His - Leu - Ile - Gly - Asp - Pro - Ser - Lys - Gln - Asn - et Ala - Thr - Ser - Ser - Pro - Leu - Tyr - Leu - Ala - , qui est active dans des analyses standards pour la lymphotoxine humaine et qui est sensiblement homogène par rapport à HPLC accomplie comme décrit dans la présente description; et les sels et dérivés des chaînes secondaires de résidus d'acide aminé ou les groupes N- et C-terminaux.

14. Protéine caractérisée en ce qu'elle a les séquences partielles d'acides aminés His - Ser - Thr - Leu - Lys - Pro - Ala - Ala - His - Leu - Ile - Gly - Asp - Pro - Ser - Lys - Gln - Asn - et Ala - Thr - Ser - Ser - Pro - Leu - Tyr - Leu - Ala - , qui est active dans des analyses standards pour la lymphotoxine humaine et qui est sensiblement homogène par rapport à SDS-PAGE accomplie comme décrit ici dans la description; et les sels et dérivés des chaînes secondaires de résidus d'acide aminé ou les groupes N- ou C-terminaux.

15. Protéine purifiée et non dérivée selon l'une quelconque des revendications 10 à 14.

16. Protéine selon l'une quelconque des revendications 10 à 15 sous une forme acceptable en pharmacie.

17. Protéine selon l'une quelconque des revendications 10 à 15 sous une forme non radioactive.

18. Composition de matière qui comprend un produit de digestion tryptique de la protéine selon l'une quelconque des revendications 10 à 17, où un fragment tryptique a la séquence partielle d'acides aminés His - Ser - Thr - Leu - Lys - Pro - Ala - Ala - His - Leu - Ile - Gly - Asp - Pro - Ser - Lys - Gln - Asn - et un autre fragment a la séquence partielle d'acides aminés Ala - Thr - Ser - Ser - Pro - Leu - Tyr - Leu - Ala - .

19. Composition pharmaceutique utile pour le traitement des tumeurs chez des êtres humains qui comprend une quantité thérapeutiquement efficace d'un composé selon l'une quelconque des revendications 10 à 18 en mélange avec un excipient acceptable en pharmacie.

**Revendications pour l'Etat Contractant: AT**

1. Procédé pour la préparation de lymphotoxine humaine, lequel procédé consiste à:

(a) obtenir un extrait, produit surnageant ou filtrat d'une culture de cellules qui produit la lymphotoxine;

(b) traiter ledit extrait, produit surnageant ou filtrat au moyen d'un absorbant de lectine de lentille;

(c) éluer sélectivement la lymphotoxine de l'absorbant.

2. Procédé de la revendication 1 qui comprend, avant l'étape (b), l'étape additionnelle de focalisation isoélectrique.

3. Procédé de la revendication 1 ou la revendication 2 qui comprend, avant ou après l'étape de focalisation isoélectrique (si elle est présente), l'étape additionnelle de chromatographie sur DEAE-cellulose.

4. Procédé de préparation de lymphotoxine, lequel procédé consiste à:

(a) concentrer l'extrait, produit surnageant ou filtrat d'une culture de cellules qui produit la lymphotoxine et dialyser le concentrat pour obtenir un dialysat;

(b) soumettre le dialysat à une chromatographie sur DEAE-cellulose;

(c) soumettre la fraction contenant la lymphotoxine de l'étape (b) à une concentration;

(d) soumettre le résultat de (c) à une focalisation isoélectrique pour obtenir une fraction contenant la lymphotoxine;

(e) soumettre la fraction contenant la lymphotoxine de l'étape (d) à une chromatographie à la lectine de lentille;

(f) récupérer la lymphotoxine de l'étape (e).

5. Procédé de la revendication 4 où la dialyse à l'étape (a) est accomplie en utilisant une feuille de polysulfone.

6. Procédé de la revendication 4 ou la revendication 5 où la lymphotoxine de l'étape (f) est obtenue en éluant sélectivement avec une solution contenant du mannose ou du galactose.

7. Procédé selon l'une quelconque des revendications précédentes où la culture de cellules est d'une lignée de cellules de lymphoblastoïde humain.

8. Procédé selon la revendication 7 où la lignée de cellules est RPMI 1788 (ATCC No. CCL 156).

9. Procédé selon l'une quelconque des revendications précédentes où la culture de cellules a été développée dans un milieu sans sérum.

10. Procédé qui comprend la production d'une lymphotoxine humaine ayant les séquences partielles d'acides aminés His - Ser - Thr - Leu - Lys - Pro - Ala - Ala - His - Leu - Ile - Gly - Asp - Pro - Ser - Lys - Gln - Asn - et Ala - Thr - Ser - Ser - Pro - Leu - Tyr - Leu - Ala - , sensiblement sans impuretés; et les sels et dérivés des chaînes secondaires de résidus d'acide aminé ou les groupes N- ou C-terminaux.

11. Procédé qui consiste à produite la lymphotoxine humaine ayant les séquences partielles d'acides aminés His - Ser - Thr - Leu - Lys - Pro - Ala - Ala - His - Leu - Ile - Gly - Asp - Pro - Ser - Lys - Gln - Asn - et Ala - Thr - Ser - Ser - Pro - Leu - Tyr - Leu - Ala - , avec une activité spécifique supérieure à $10 \times 10^6$ unités/mg; et les sels et dérivés des chaînes secondaires de résidus d'acide aminé ou les groupes N- ou C-terminaux.

12. Procédé qui consiste à produire la lymphotoxine humaine ayant les séquences partielles d'acides aminés His - Ser - Thr - Leu - Lys - Pro - Ala - Ala - His - Leu - Ile - Gly - Asp - Pro - Ser - Lys - Gln - Asn - et Ala - Thr - Ser - Ser - Pro - Leu - Tyr - Leu - Ala - , avec une activité spécifique supérieure à $100 \times 10^6$ unités/mg; et les sels et dérivés des chaînes secondaires de résidus d'acide aminé ou les groupes N- ou C-terminaux.

13. Procédé qui consiste à produire une protéine ayant les séquences partielles d'acides aminés His - Ser - Thr - Leu - Lys - Pro - Ala - Ala - His - Leu - Ile - Gly - Asp - Pro - Ser - Lys - Gln - Asn et Ala - Thr - Ser - Ser - Pro - Leu - Tyr - Leu - Ala - , qui est active dans des analyses standards pour la lymphotoxine humaine et qui est sensiblement homogène par rapport à une HPLC accomplie comme décrit ici dans la description; et des sels et dérivés des chaînes secondaires de résidus d'acide aminé ou les groupes N- ou C-terminaux.

14. Procédé qui consiste à produire une protéine ayant les séquences partielles d'acides aminés His - Ser - Thr - Leu - Lys - Pro - Ala - Ala - His - Leu - Ile - Gly - Asp - Pro - Ser - Lys - Gln - Asn - et Ala - Thr - Ser - Ser - Pro - Leu - Tyr - Leu - Ala - , qui est active dans des analyses standards pour la lymphotoxine humaine et qui est sensiblement homogène par rapport à SDS-PAGE accomplie comme décrit dans la description; et des sels et dérivés des chaînes secondaires de résidus d'acide aminé ou les groupes N- ou C-terminaux.

15. Procédé selon l'une quelconque des revendications 10 à 14 où la protéine est produite sous une forme purifiée et non dérivée.

16. Procédé selon l'une quelconque des revendications 10 à 15 où la protéine est produite sous une forme acceptable en pharmacie.

17. Procédé selon l'une quelconque des revendications 10 à 15 où la protéine est produite sous une forme non radioactive.

18. Procédé qui consiste à produire une composition de matière par digestion tryptique de la protéine selon l'une quelconque des revendications 10 à 17, où un fragment tryptique à la séquence partielle d'acides aminés His - Ser - Thr - Leu - Lys - Pro - Ala - Ala - His - Leu - Ile - Gly - Asp - Pro - Ser - Lys - Gln - Asn - et un autre fragment a la séquence partielle d'acides aminés Ala - Thr - Ser - Ser - Pro - Leu - Tyr - Leu - Ala - .

Cell Culture (Human Lymphoblastoid 1788)

Step 1 | Prefilter

FILTRATE

Step 2 | Concentrate and Dialyze with Pellicon

CONCENTRATE (I)

Step 3 | DE-52 DEAE Cellulose Chromatography

ACTIVE FRACTION (I)

Step 4 | Concentrate with Amicon

CONCENTRATE (II)

Step 5 | Preparative Isoelectric Focusing

pH 5.5 - 6.5 FRACTION

Step 6 | Lentil-Lectin Chromatography

ACTIVE FRACTION (II)
(Lymphotoxin)

*Fig. 1*

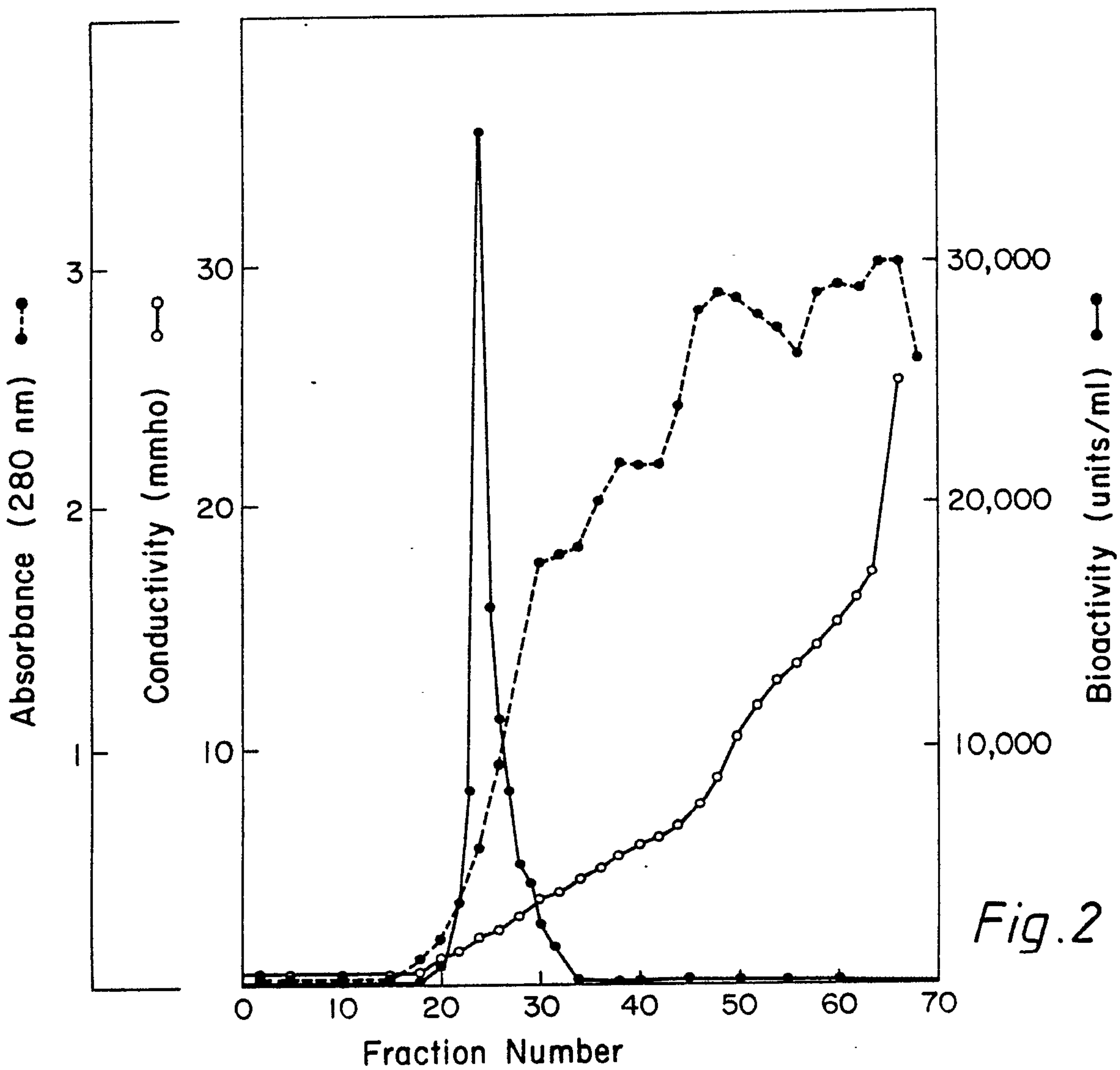
Absorbance (280 nm)
Conductivity (mmho)
Bioactivity (units/ml)
Fraction Number
3
2
1
30
20
10
30,000
20,000
10,000
0
10
20
30
40
50
60
70

Fig.2

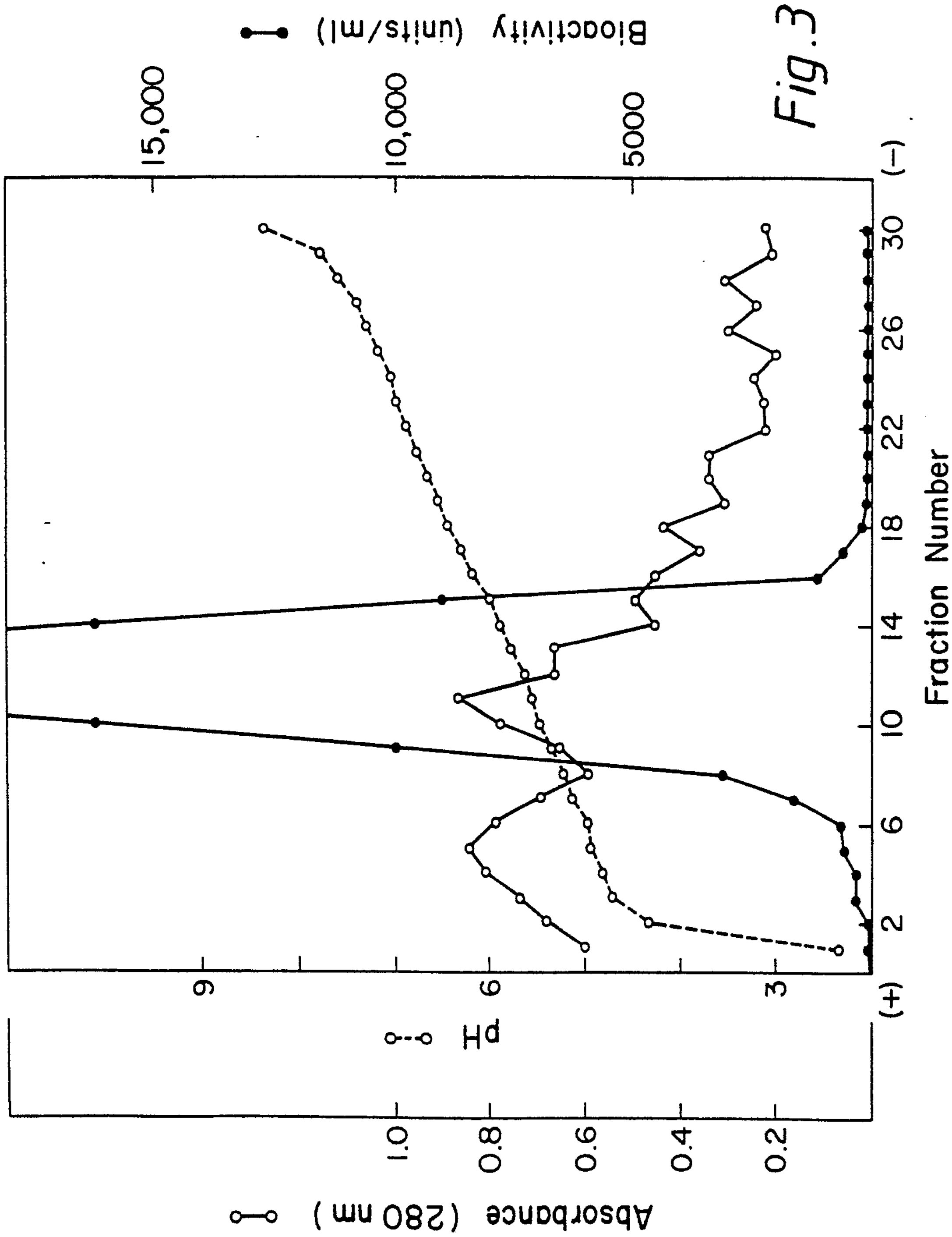
Bioactivity (units/ml)
15,000
10,000
5000
(–)
(+)
Absorbance (280 nm)
1.0
0.8
0.6
0.4
0.2
pH
9
6
3
2
6
10
14
18
22
26
30
Fraction Number

Fig. 3

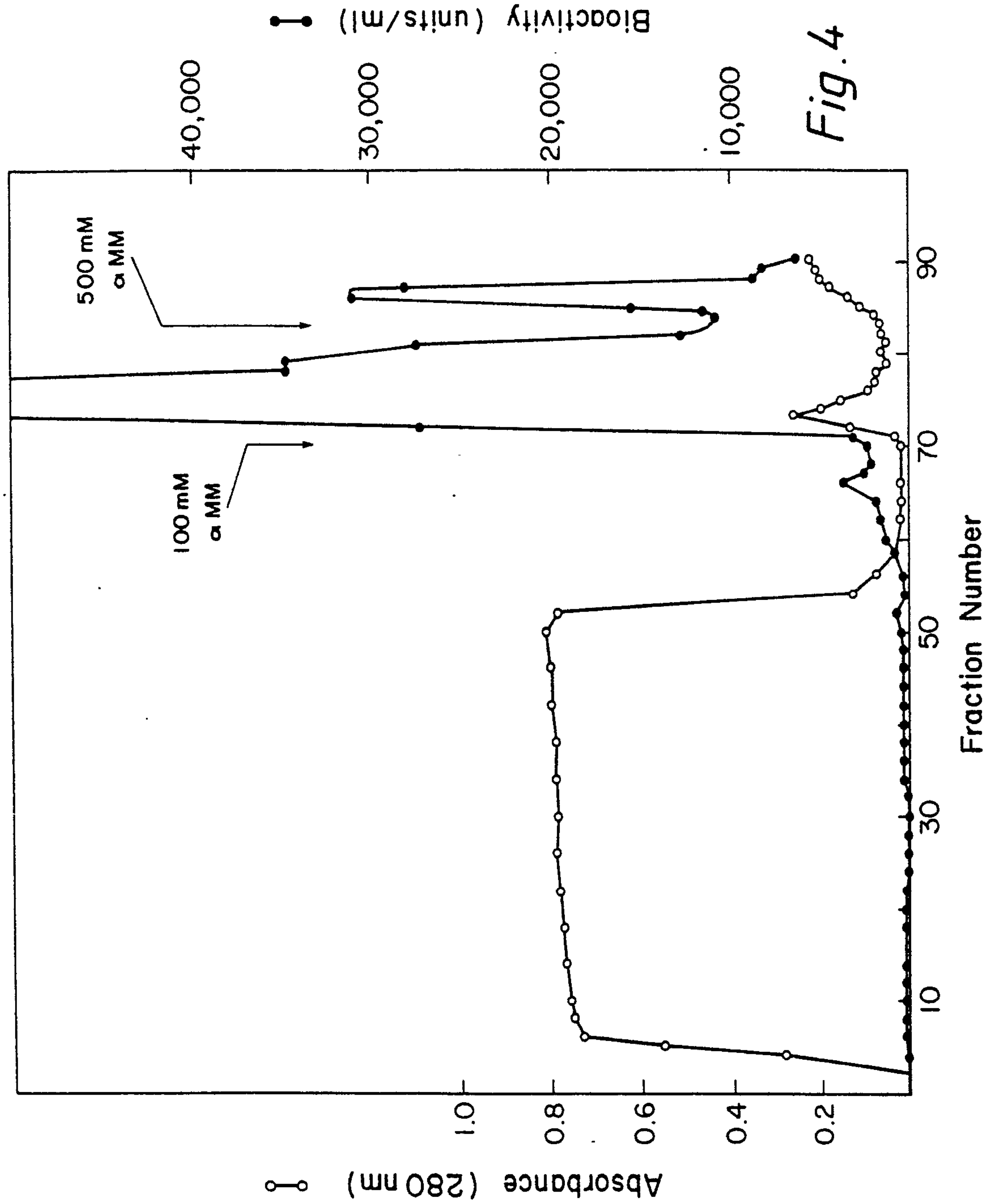
Bioactivity (units/ml)
40,000
30,000
20,000
10,000
500 mM
α MM
100 mM
α MM
Fraction Number
10
30
50
70
90
1.0
0.8
0.6
0.4
0.2
Absorbance (280 nm)

Fig. 4

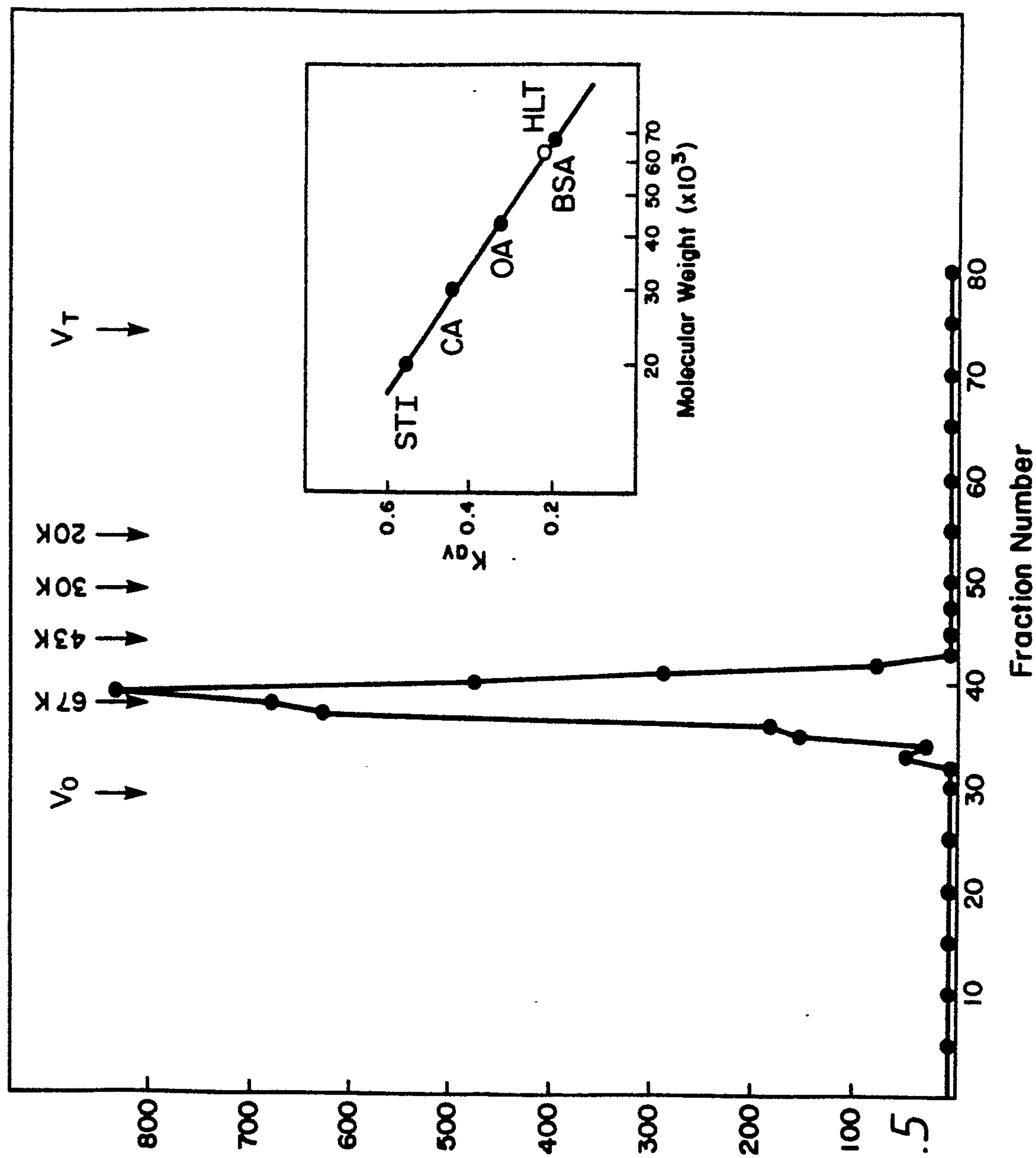

Bioactivity (units/ml)
800
700
600
500
400
300
200
100
Fraction Number
10
20
30
40
50
60
70
80
$V_O$
67 K
43K
30K
20K
$V_T$
$K_{av}$
0.6
0.4
0.2
Molecular Weight ($x10^3$)
20
30
40
50
60
70
STI
CA
OA
BSA
HLT


Fig.5

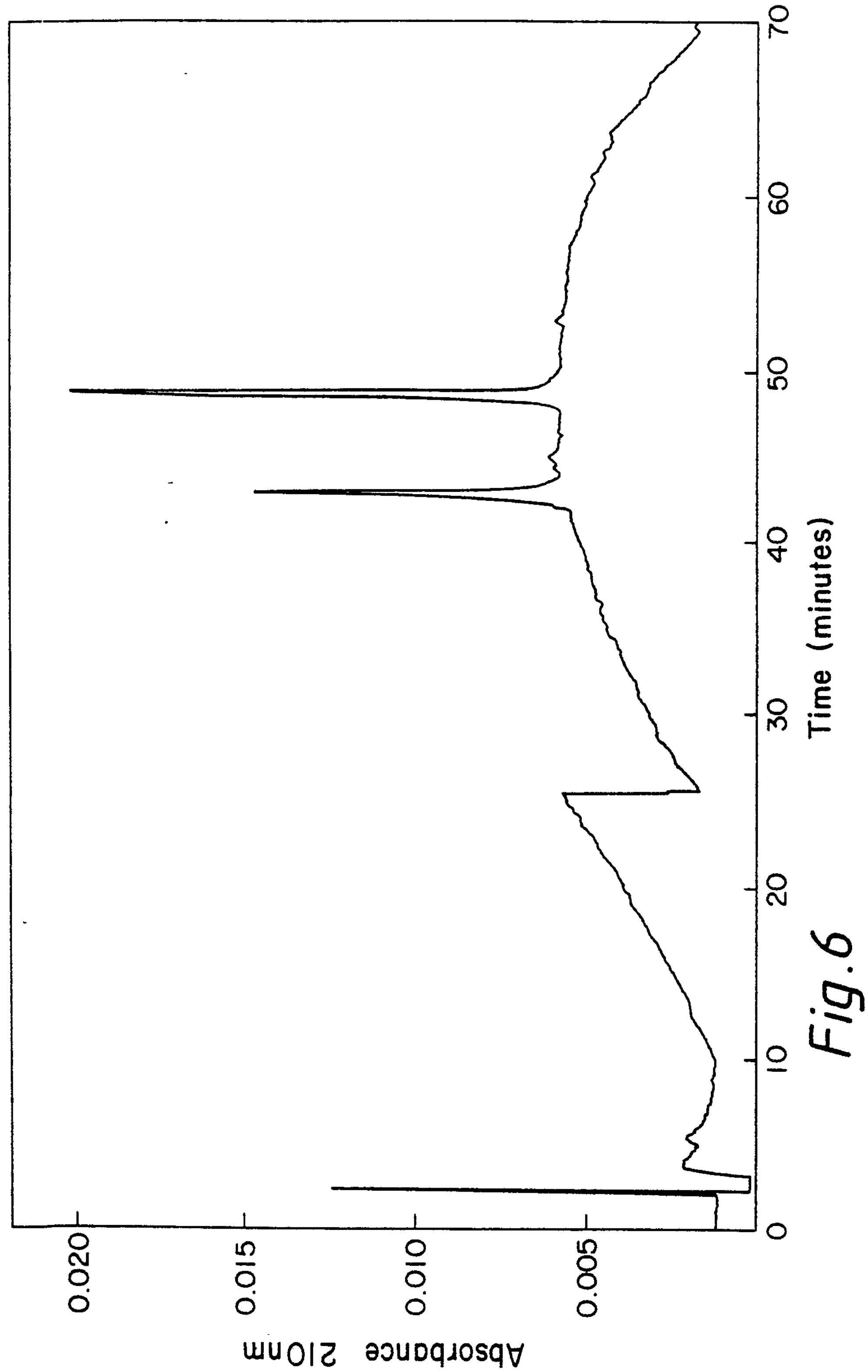

Absorbance 210nm
0.020
0.015
0.010
0.005
0
10
20
30
40
50
60
70
Time (minutes)


Fig.6

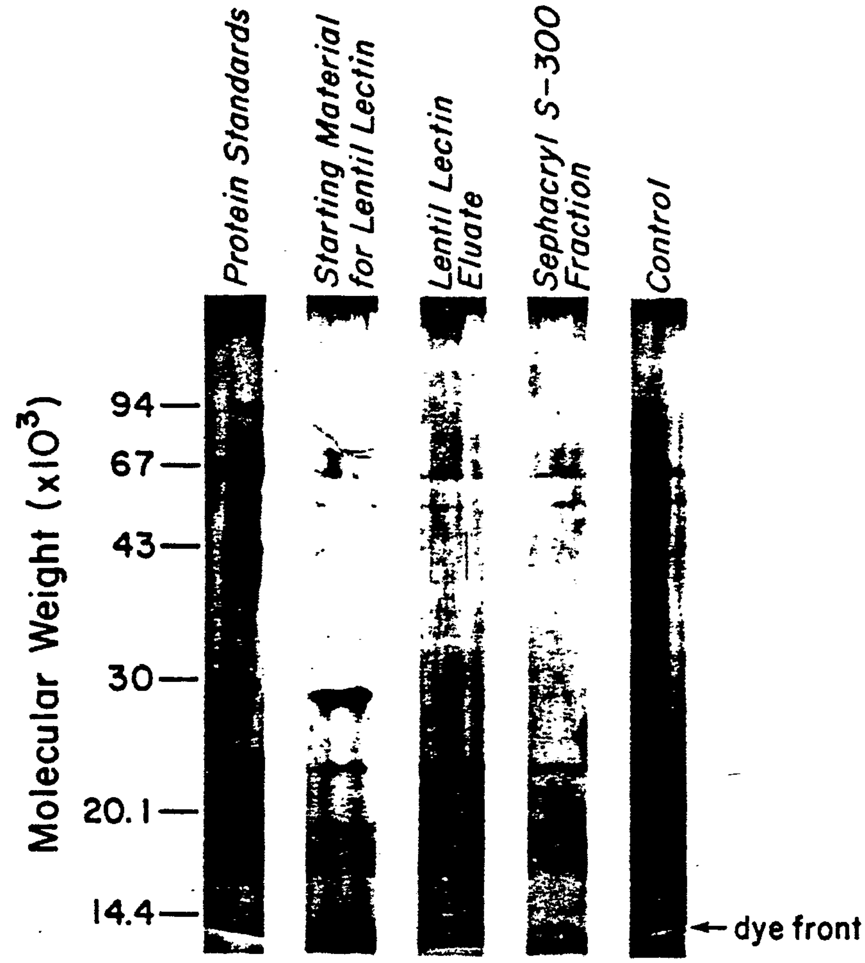
Protein Standards
Starting Material for Lentil Lectin
Lentil Lectin Eluate
Sephacryl S-300 Fraction
Control
Molecular Weight (x10³)
94
67
43
30
20.1
14.4
dye front

Fig. 7

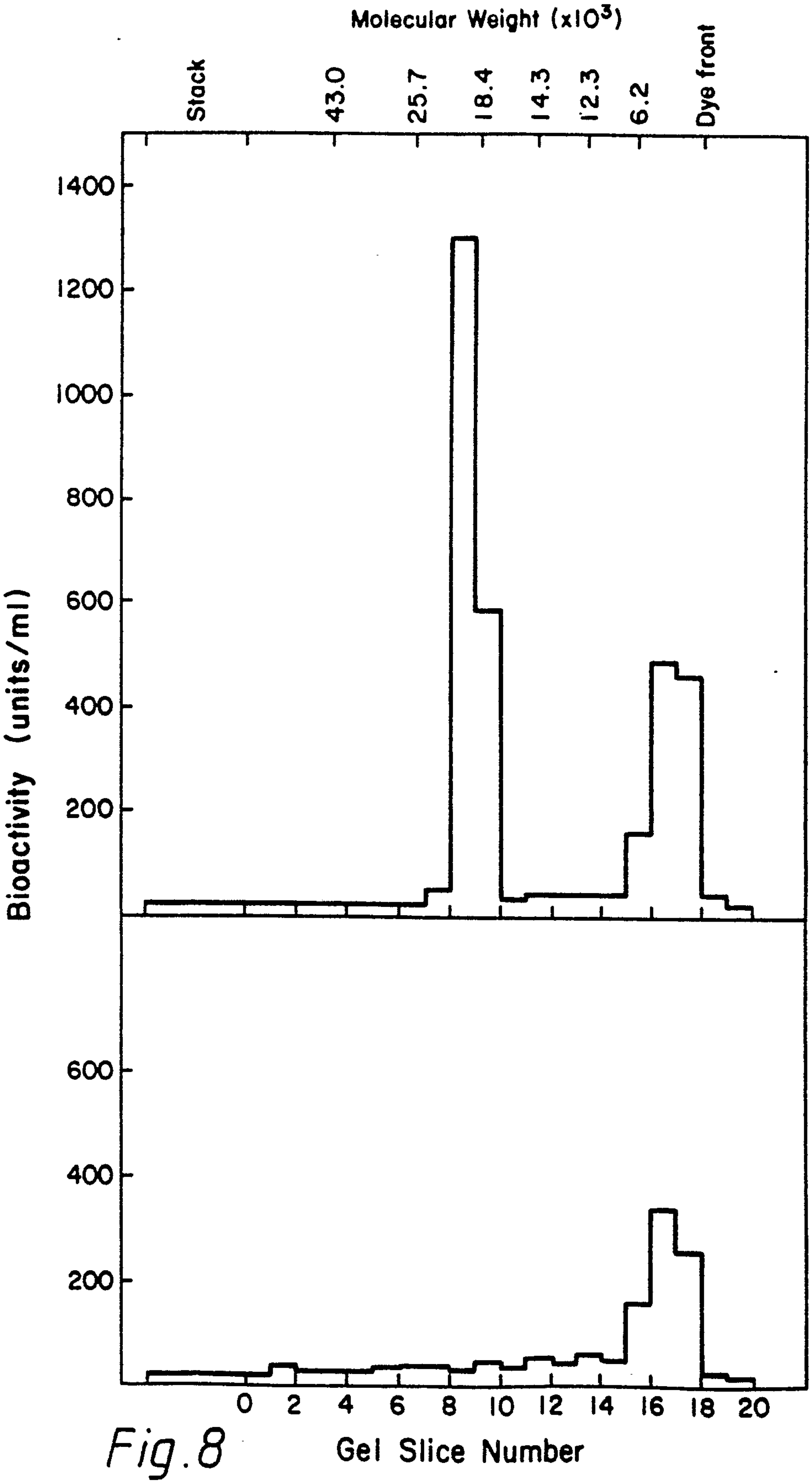
Molecular Weight (x10³)
Stack
43.0
25.7
18.4
14.3
12.3
6.2
Dye front
Bioactivity (units/ml)
1400
1200
1000
800
600
400
200
600
400
200
0
2
4
6
8
10
12
14
16
18
20
Gel Slice Number

Fig.8

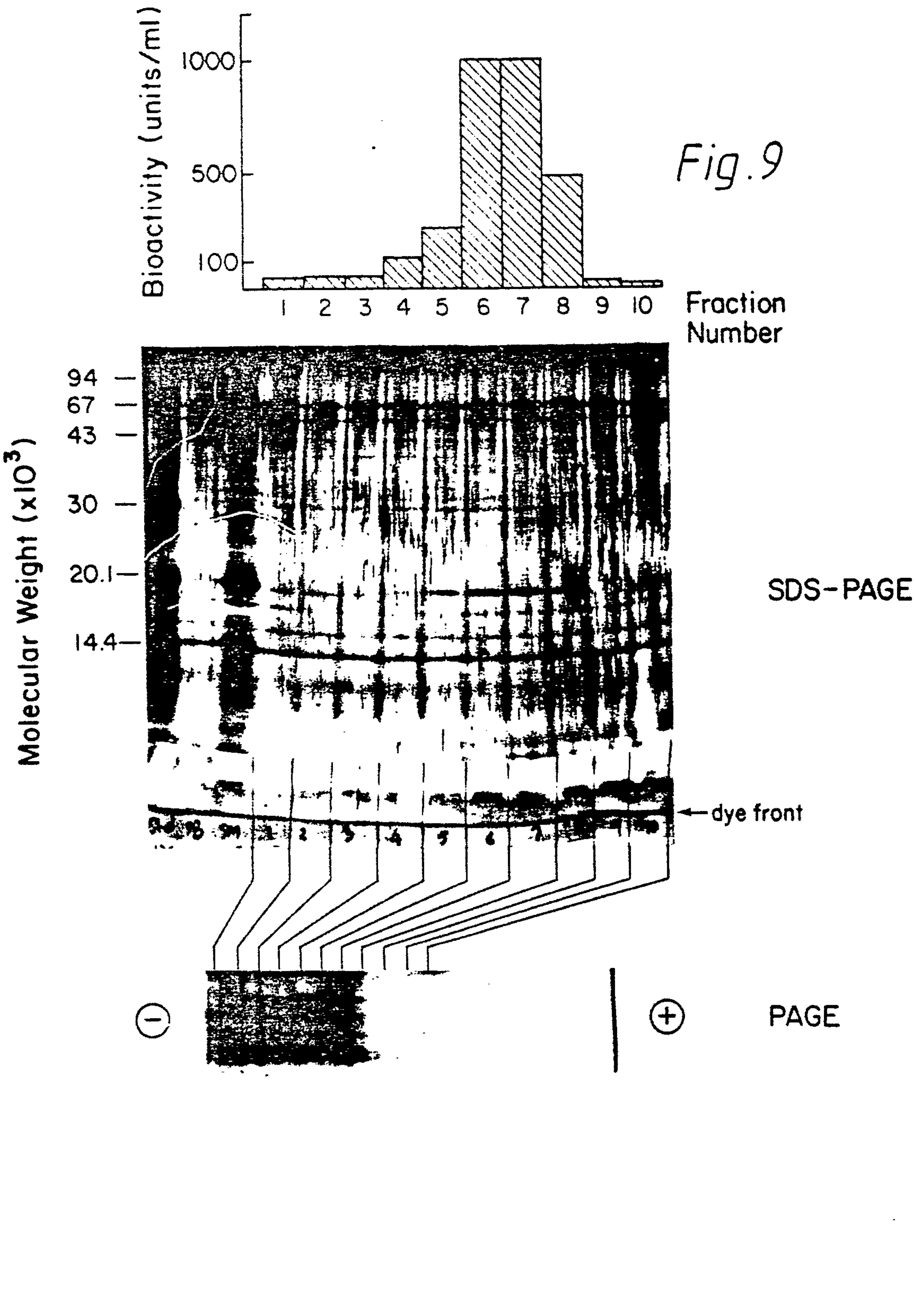
Bioactivity (units/ml)
1000
500
100
1 2 3 4 5 6 7 8 9 10
Fraction Number
Molecular Weight (x10^3)
94
67
43
30
20.1
14.4
SDS-PAGE
dye front
PAGE

Fig.9

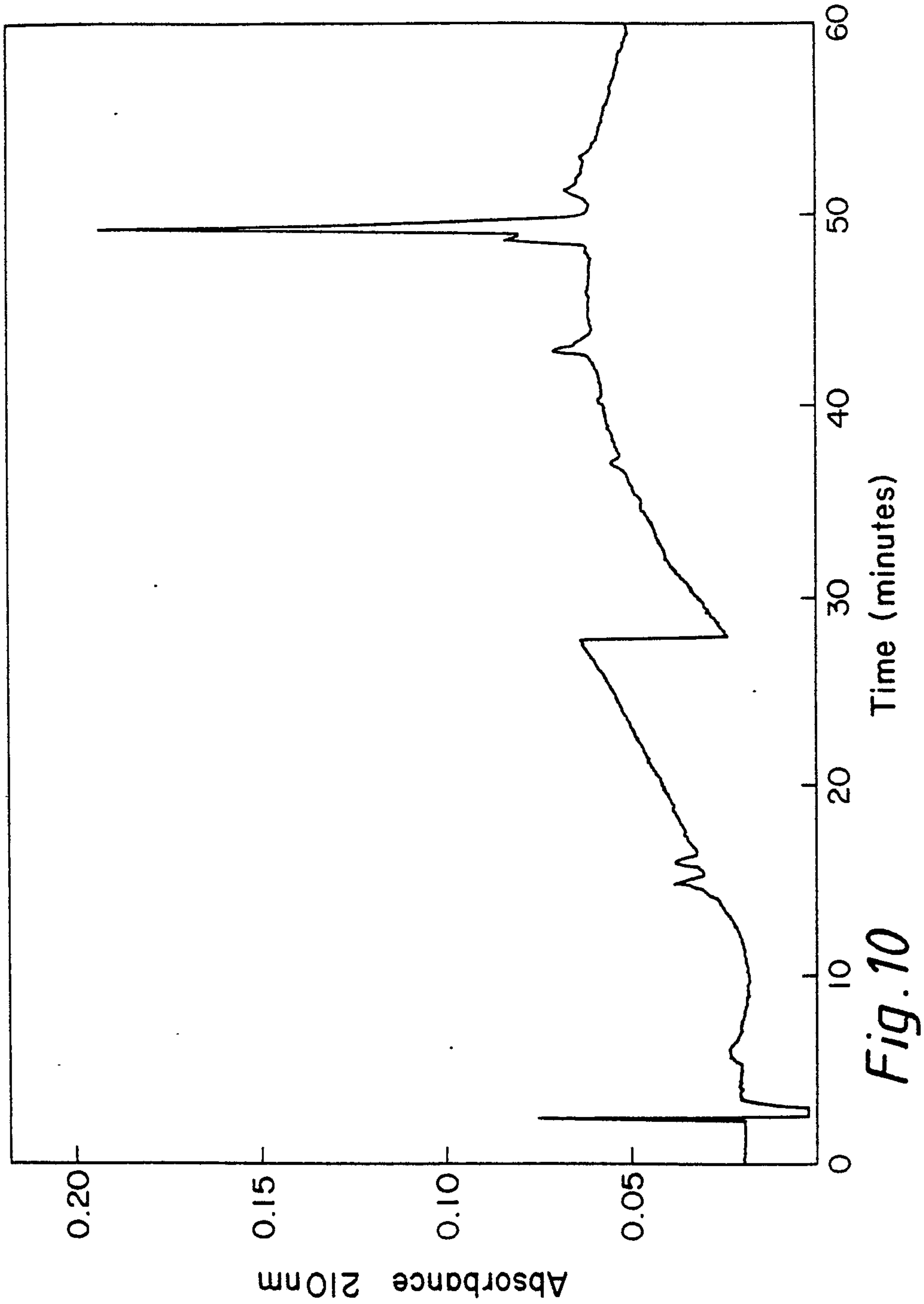

Absorbance 210nm
0.20
0.15
0.10
0.05
Time (minutes)
0
10
20
30
40
50
60


Fig. 10

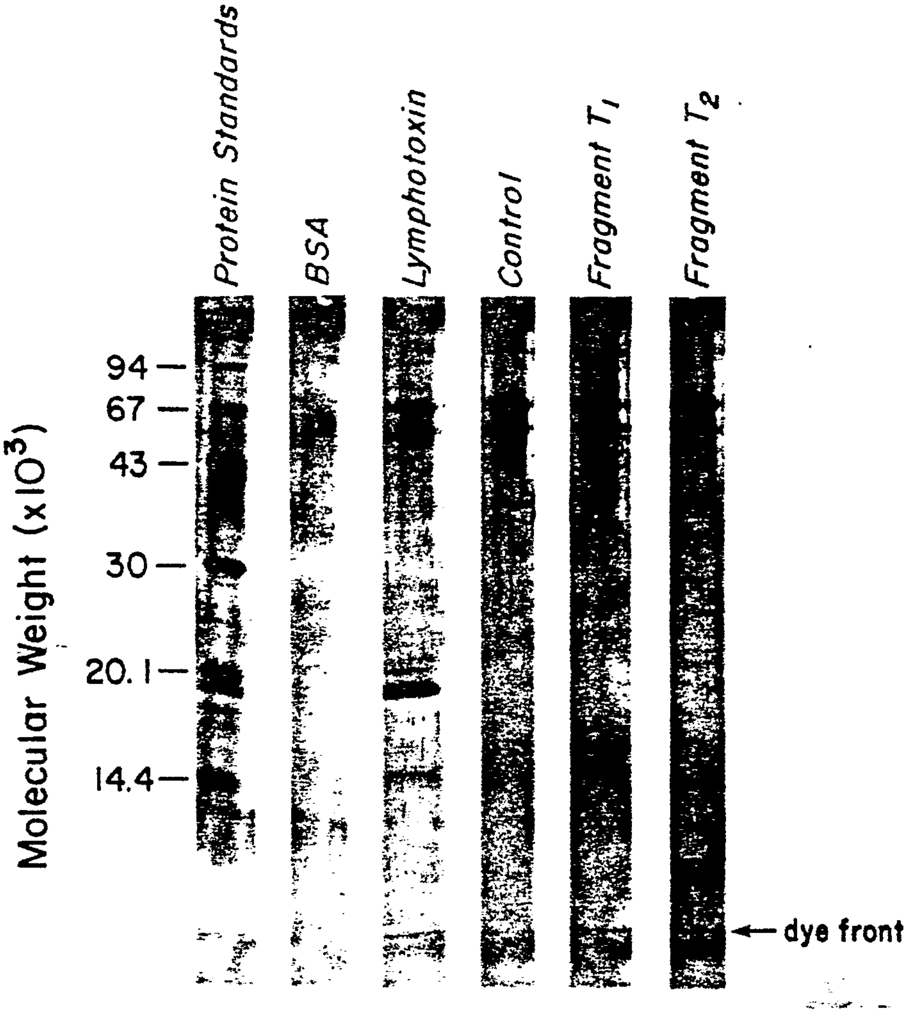
Protein Standards
BSA
Lymphotoxin
Control
Fragment $T_1$
Fragment $T_2$
Molecular Weight (x10³)
94
67
43
30
20.1
14.4
dye front

Fig. 11

PARTIAL AMINO ACID SEQUENCE OF HUMAN LYMPHOTOXIN

<u>Tryptic Fragment $T_2$ (5K)</u>

| | | | | 5 | | | | | 10 |
|---|---|---|---|---|---|---|---|---|---|
| Ala - | Thr - | Ser - | Ser - | Pro - | Leu - | Tyr - | Leu - | Ala - | Cys - |

| | | | | 15 | | | | | 20 |
|---|---|---|---|---|---|---|---|---|---|
| - Glu - | Val - | Gln - | Leu - | Phe - | Cys - | Cys - | Gln - | Tyr - | Pro - |

| | | | | 25 | |
|---|---|---|---|---|---|
| - Phe - | X - | Val - | X - | X - | . . . . . . . . . . |

<u>Tryptic Fragment $T_1$ (15K)</u>

| | | | | 5 | | | | | 10 |
|---|---|---|---|---|---|---|---|---|---|
| His - | Ser - | Thr - | Leu - | Lys - | Pro - | Ala - | Ala - | His - | Leu - |
| (Ser) | | (Leu) | (Lys) | (Pro) | (Ala) | | | | |
| (Ala) | | | | | | | | | |

| | | | | 15 | | | | | 20 |
|---|---|---|---|---|---|---|---|---|---|
| - Ile - | Gly - | Asp - | Pro - | Ser - | Lys - | Gln - | Asn - | (Cys) - | Leu - |

| | | | | 25 | | | | | 30 |
|---|---|---|---|---|---|---|---|---|---|
| - Leu - | Trp - | Arg - | Ala - | Asn - | (Ser) - | Asp - | Arg - | Ala - | Phe - |

| | | | | 35 | | | | | 40 |
|---|---|---|---|---|---|---|---|---|---|
| - Leu - | Glu - | (Ser) - | Gly - | Phe - | Ser - | Leu - | Asp - | X - | Asn - |
| | | | | | (Cys) | | (Thr) | | (Thr) |
| | | | | | (Phe) | | (Ser) | | (Ser) |
| | | | | | | | (His) | | (His) |
| | | | | | | | (Arg) | | (Arg) |
| | | | | | | | (Cys) | | (Cys) |

| | | | | 45 |
|---|---|---|---|---|
| - Val - | Leu - | X - | X - | X - |
| (Asn) | (Val) | | | |

*Fig. 12*